# EUROPÄISCHE PATENTANMELDUNG

(11) **EP 2 338 562 A1**
(43) Veröffentlichungstag der Anmeldung: **29.06.2011**
(21) Anmeldenummer: 10192889.3
(22) Anmeldetag: 29.11.2010
(51) Int. Cl.: A61N 1/37, G01R 33/28

(54) **MRT-Lorentz-Vibrator**

(30) Priorität: 22.12.2009 US 288860 P
(71) Anmelder: BIOTRONIK CRM Patent AG, 6341 Baar (CH)
(72) Erfinder: Dörr, Thomas, 12437, Berlin (DE); Weiss, Ingo, 12435, Berlin (DE)
(74) Vertreter: Lindner-Vogt, Karin L.

(57) **Zusammenfassung**

Die Erfindung bezieht sich auf eine Vorrichtung und ein Verfahren zum Erkennen von elektromagnetischen Feldern, speziell solche Felder, wie sie bei bildgebenden Kernspintomographieuntersuchungen (im folgenden MRT oder MRI) auftreten. Insbesondere bezieht sie sich auf ein implantierbares medizinisches Gerät (IMD) beinhaltend eine Einheit zur Detektion von MRT-Aktivität, wobei die Einheit zur Detektion von MRT-Aktivität mindestens einen Schwingungswandler beinhaltet, der durch ein MRT-Gerät hervorgerufene Vibrationen und/oder Schwingungen in ein elektrisches und/oder optisches Signal umwandelt und die Einheit zur Detektion vom MRT-Aktivität auf Basis dieses Signals eine MRT-Aktivität erkennt.

## Beschreibung

Die Erfindung bezieht sich auf eine Vorrichtung und ein Verfahren zum Erkennen von elektromagnetischen Feldern, speziell solche Felder, wie sie bei bildgebenden Kernspintomographie- (im folgenden MRT- oder MRI-) Geräten auftreten.

Obwohl MRI-Untersuchungen einen immer höheren Stellenwert in der diagnostischen Medizin erhalten, ist ein Teil der Patienten für MRI-Untersuchungen kontraindiziert. Eine solche Kontraindizierung kann durch ein mindestens teilweise implantiertes medizinisches Gerät (im Folgenden auch Implantat oder IMD) gegeben sein.

Um MRI-Untersuchungen dennoch zu ermöglichen, sind verschiedene Ansätze, die sich entweder auf die Durchführung der MRI-Untersuchung oder auf das implantierbare medizinische Gerät beziehen, bekannt.

Unter anderem sind Technologien zur Erkennung von Magnetfeldern bekannt, die auf herkömmlichen Verfahren zur Magnetfelddetektion beruhen. So beschreibt die US 2008/0154342 ein Verfahren unter Ausnutzung eines GMR-Sensors (Giant Magnetic Resistance), um problematische Magnetfelder von MRT-Geräten zu erkennen. Diese technologischen Ansätze sind allerdings wenig spezifisch und erzeugen einen erhöhten Energiebedarf, was zu einer kürzen Betriebsdauer bei gleichen Energiereserven führt.

Auch sind Systeme im Stand der Technik bekannt, die die Änderung von Schwingungsparametem zur Bestimmung von Änderungen von Magnetfeldstärken für periodisch schwingenden Systeme verwenden, wie es in der US 2006/0265139 beschrieben wird. Solche Systeme sind aufgrund der Rahmenbedingungen, wie zum Beispiel die periodische Schwingung, nicht für die Detektion von Magnetfeldern im Umfeld von Implantaten geeignet.

Aufgabe der Erfindung ist es, eine Vorrichtung und eine Methode für medizinische Geräte und implantierbare medizinische Geräte bereitzustellen, die die Nachteile des Stands der Technik beheben und eine zuverlässige Erkennung von elektromagnetischen Feldern erlaubt.

Die Aufgabe wird durch ein implantierbares medizinisches Gerät (IMD) mit den Merkmalen des Anspruchs 1 und ein Verfahren nach Anspruch 15 gelöst.

Das implantierbare medizinische Gerät (IMD) beinhaltet dabei eine Einheit zur Detektion von MRT-Aktivität, wobei die Einheit zur Detektion von MRT-Aktivität mindestens einen Schwingungswandler beinhaltet, der durch ein MRT-Gerät hervorgerufene Vibrationen und/oder Schwingungen in ein elektrisches und/oder optisches Signal umwandelt und die Einheit zur Detektion vom MRT-Aktivität auf Basis dieses Signals eine MRT-Aktivität erkennt.

Unter MRT-Aktivität ist in diesem Zusammenhang die bei der Nutzung eines MRT-Gerät auftretenden elektromagnetischen Störfelder und andere mechanische Effekte zu verstehen. Diese umfassen zum Beispiel, aber nicht ausschließlich, statische elektromagnetische Felder, elektromagnetische Gradientenfelder und/oder Radiofrequenzfelder, aber auch mechanische Vibrationen, hervorgerufen durch elektromagnetische Wechselfelder und/oder mechanische bewegte Objekte, wie, aber nicht beschränkt, auf den beweglichen Untersuchungstisch.

Im Zusammenhang mit den elektromagnetischen Feldern und/oder beweglichen Objekten kommt es sowohl am MRT-Gerät zu charakteristischen Vibrationen und/oder Schwingungen, als auch im IMD. Die Vibrationen und/oder Schwingungen im IMD können dabei sowohl durch eine mechanische Ankopplung auf das IMD übertragen werden, als auch durch elektromagnetische Felder induziert werden oder durch elektromagnetische Felder in Komponenten induziert werden. Diese Komponenten können herkömmliche Komponenten eines IMD sein oder aber speziell für die Erkennung von Schwingungen und/oder Vibrationen ausgelegte Komponenten sein.

Bevorzugt wird, dass basierend auf der Erkennung einer MRT-Aktivität ein Betriebsmodus für das IMD ausgewählt wird, der einen sicheren Betrieb unter MRT-Aktivität erlaubt.

Auch wird bevorzugt, dass die Vibrationen durch Lorentz-Kräfte über elektromagnetische Felder vom MRT-Gerät auf den Schwingungswandler übertragen werden und/oder dass die Vibrationen durch Lorentz-Kräfte über elektromagnetische Felder vom MRT-Gerät auf das IMD und von dort auf den Schwingungswandler übertragen werden und/oder dass die Vibrationen im MRT-Gerät entstehen und diese mechanisch auf den Schwingungswandler übertragen werden, wobei der Schwingungswandler für unterschiedliche Übertragungsarten auslegbar ist und/oder separate Schwingungswandler für die jeweils unterschiedlichen Übertragungsarten vorsehbar sind.

Separate Schwingungswandler weisen zumindest unterschiedlichen Empfindlichkeiten für die Detektion der unterschiedlichen Schwingungen und/oder Vibrationen auf.

Ebenfalls bevorzugt wird, dass zumindest ein Schwingungswandler auf das für MRT-Geräte typische Frequenzspektrum der MRT-Geräusche abgestimmt ist.

Als MRT-Geräusche werden alle Geräusche bezeichnet, die typischerweise bei der Benutzung eines MRT-Gerätes entstehen, wie durch Lorentz-Kräfte im MRT-Gerät induzierte Vibrationen und/oder Schwingungen, und/oder durch automatisierte mechanische Bewegungen hervorgerufen Schwingungen und/oder Vibrationen.

Des Weiteren wird bevorzugt, dass zumindest ein Schwingungswandler auch als Beschleunigungssensor nutzbar ist und dieser Beschleunigungssensor für die ratenadaptive Stimulation eines Herzschrittmachers und/oder Defibrillators/Kardioverters und/oder kardialen Resynchronisationsgerätes verwendbar ist.

Auch wird bevorzugt, dass der oder die Schwingungswandler in einem heliumdichten und/oder evakuierten Gehäuse untergebracht sind. Die Heliumdichtigkeit und die Evakuierung verhindern und/oder verringern dabei das spätere Eindringen von Helium in das Gehäuse und minimieren somit Dämpfungseffekte auf den Schwingungswandler.

Ebenfalls bevorzugt wird, dass zumindest ein Schwingungswandler nach dem Prinzip der Lorentzkraft immer nur dann in Schwingungen versetzbar ist, wenn gleichzeitig ein Magnetfeld mit einer vorbestimmbaren Mindestfeldstärke vorhanden ist und durch magnetische Wechselfelder Wirbelströme induziert werden.

Bevorzugt wird auch, dass zumindest ein Schwingungswandler mechanisch mit einem Hochspannungstransformator verbunden ist.

Hochspannungstransformatoren sind in Geräten wie Defibrillatoren/Kardiovertern vorsehbar.

Auch wird bevorzugt, dass zumindest ein Schwingungswandler mit einer Programmierspule des IMD verbindbar ist, so dass die Programmierspule durch MRT-Aktivität in Schwingungen versetzbar ist, diese Schwingungen auf den Schwingungswandler übertragbar sind und das Resonanzverhalten der Programmierspule an typische MRT-Aktivitäten anpassbar ist.

Des Weiteren wird bevorzugt, dass zumindest ein Schwingungswandler aus einem piezoelektrischen Kern umgeben von einer Spule besteht, wobei die Spule durch Lorentzkräfte den piezoelektrischen Kern in Schwingungen versetzt und so ein piezoelektrisches Signal erzeugt, das als Signal zur Detektion von MRT-Aktivität verwendbar ist.

Bevorzugt wird auch, dass zumindest ein Schwingungswandler aus einem Beschleunigungssensor als Kern, der von einer Spule umgeben ist, besteht, wobei der Kern von der Spule durch Lorentzkräfte in Schwingungen versetzbar ist.

Ebenfalls bevorzugt wird, dass zumindest ein Schwingungswandler typische MRT-Geräusche mittels zumindest eines Mikrophons und/oder zumindest eines Beschleunigungssensor wahrnimmt, die wahrgenommenen MRT-Geräusche filterbar sind und anschließend durch Autokorrelation in einem digitalen Signalprozessor (DSP) typischen Sequenzen von MRT-Geräuschen zuordenbar sind.

Auch wird bevorzugt, dass zumindest die Signale eines Schwingungswandlers mittels eines für die MRT-Geräuscherkennung angepassten Stimmenidentifikationsverfahrens und/oder eines neuronalen Netzwerkes, wie "paralleles Vergleichen von Strukturen", verarbeitet werden.

Des Weiteren wird bevorzugt, dass zumindest eine der folgenden Maßnahmen Teil eines sicheren Betriebszustands bei MRT-Aktivität sind, das Wechseln in einen MRI-sicheren Zustand, ein verlängertes Verbleiben in einem MRI-sicheren oder gegenüber elektromagnetischen Störfeldern unempfindlichen Zustand, die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit, neben der Störung auch Informationen auf diese Weise zu übertragen und auf dem Bildschirm des MRI sichtbar zu machen, und die Abgabe einer Therapie und/oder Detektion von elektrischen Zuständen des Gewebes nur in Zeitfenstern erlaubt ist, in denen keine elektromagnetischen Störfelder erkannt werden und/oder dass eine Rekonstruktion einer Messung für die Bereiche durchgeführt wird, in denen die Detektion aufgrund von erkannten elektromagnetischen Störfeldern nicht erlaubt ist.

Bevorzugt wird auch, dass die Einheit zur Detektion MRT-Aktivität mindestens einen der folgenden Sensoren oder Indikatoren umfasst: GMR-Sensor, MagFET-Sensor, Hallsensor, elektrooptische Wandler als Indikator, die Überwachung von Batteriespannungen während Kondensatorladeprozessen als Indikator, die Detektion von RF-Feldern als Indikator, die Detektion von magnetischen Grandientenfeldern als Indikator, und die Detektion von durch elektromagnetische Felder induzierten Strömen als Indikator.

Gelöst wird die Aufgabe auch durch ein Verfahren zur Detektion von MRT-Aktivität, wobei ein Schwingungswandler durch ein MRT-Gerät hervorgerufene Vibrationen und/oder Schwingungen in ein elektrisches und/oder optisches Signal umwandelt und eine Einheit zur Detektion von MRT-Aktivität auf Basis dieses Signals eine MRT-Aktivität erkennt. Eine Kombination des Verfahrens mit den anderen Ausführungsbeispielen ist explizit möglich.

Einige Aspekte der Erfindung werden in den Figuren 1 -4 dargestellt.
Fig. 1 schematische Darstellung des Ablaufs einer MRI-Untersuchung
Fig. 2 Blockschaltbild einer erfindungsgemäßen Lösung
Fig. 3 schematische Darstellung eines IMD mit möglichen Positionen der Schwingungswandler
Fig. 4 schematischer Aufbau eines Lorentz-Sensors.

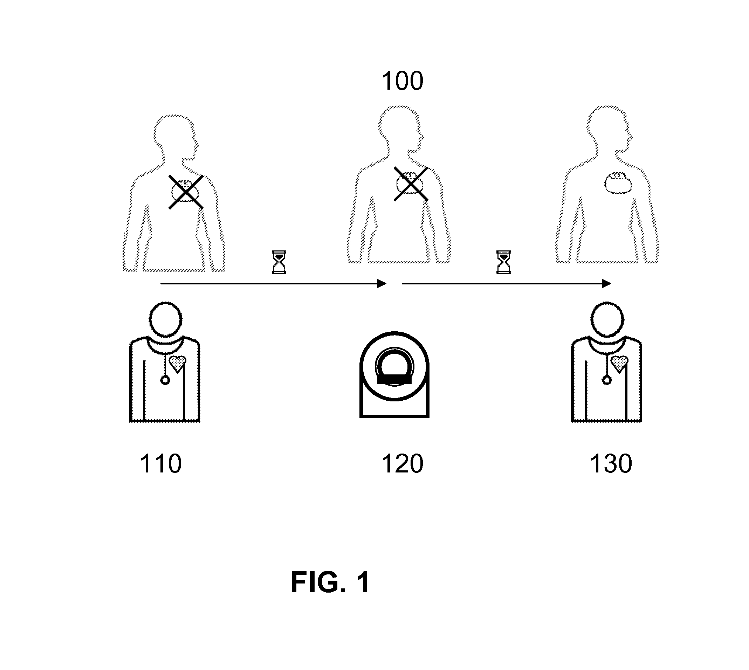

Figur 1 beschreibt den Stand der Technik, bei dem der ICD-Patient (100) vor der geplanten MRT-Untersuchung von einem Kardiologen nachgesorgt und der ICD ausgeschaltet (110) wird. Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung, in Kauf genommen.

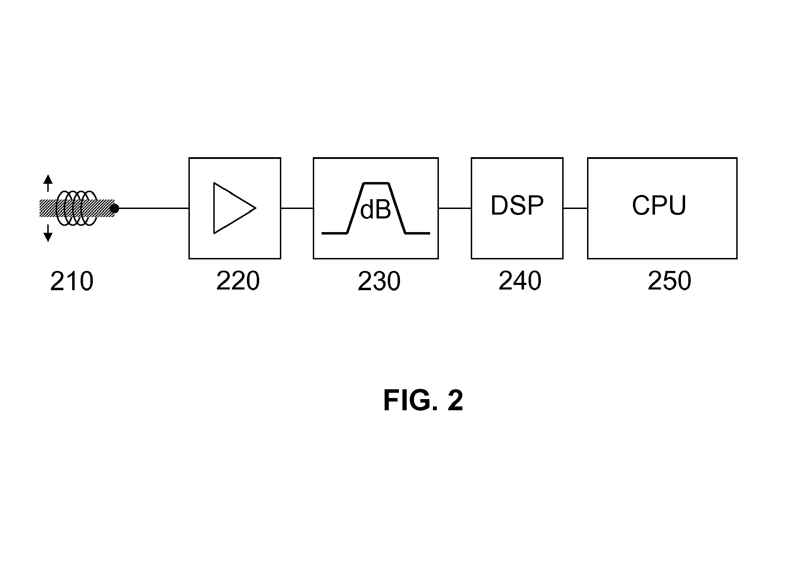

Figur 2 zeigt ein Blockschaltbild der erfindungsgemäßen Lösung zur Detektion von Schwingungen im elektronischen Implantat, wie sie von einem MRT-Gerät induziert werden. Der Schwingungswandler (210) ist mit einer Verstärker- und Digitalisierungseinheit (220) verbunden. Das digitalisierte Sensorsignal wird anschließend auf die MRT-typischen Frequenzinhalte gefiltert (230) und danach in einem digitalen Signalprozessor (240) ausgewertet. Für die Auswertung sind dabei die Autokorrelation und übliche Verfahren der Spracherkennung (speziell Stimmenidentifikation) vorsehbar. Wird eine MRT-typische Vibration und/oder Schwingung erkannt, so reagiert das Implantat mit einem verändertem Verhalten, wie das Umschalten, gesteuert über eine CPU/Steuereinheit (250), in eine vorprogrammierbare MRT-sichere Betriebsart.

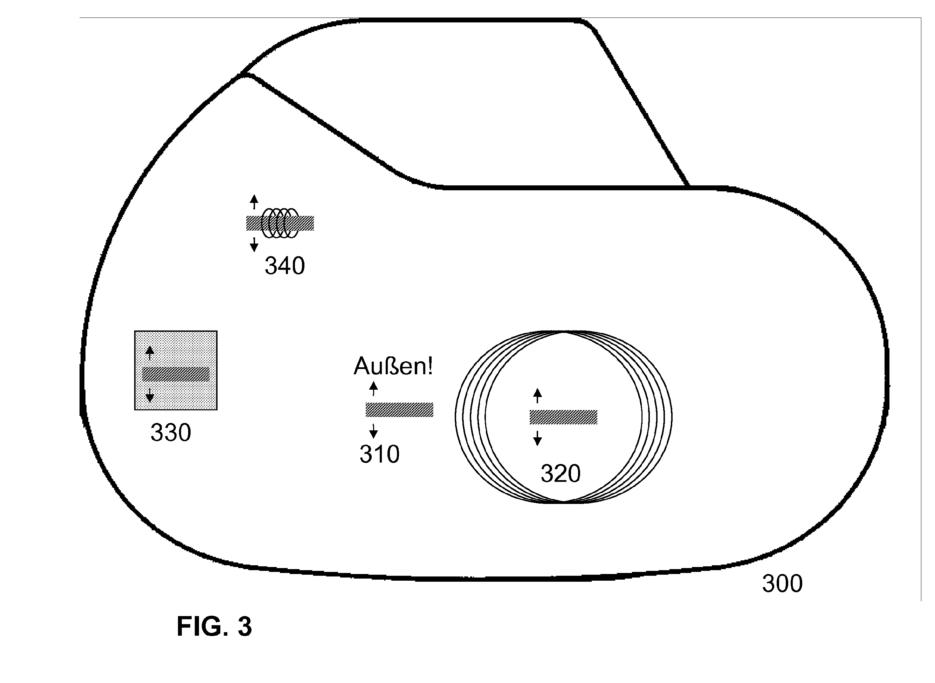

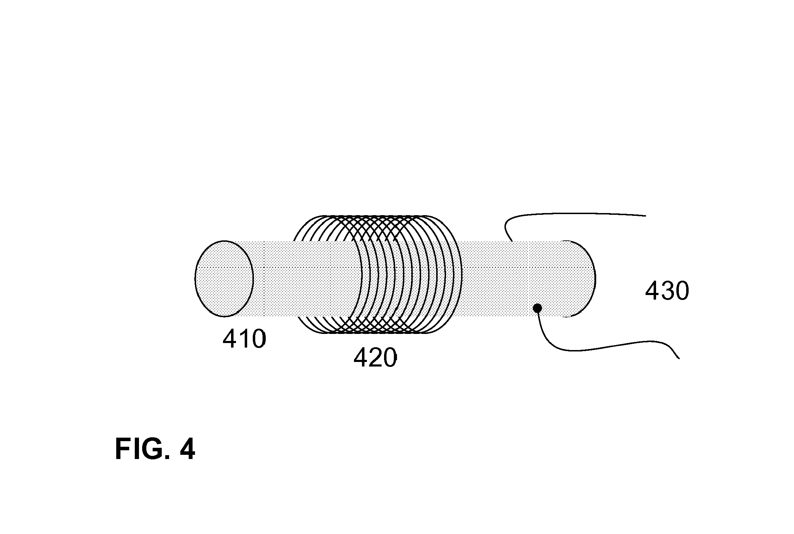

Figur 3 zeigt eine schematische Darstellung eines IMD mit möglichen Positionen der Schwingungswandler für die MRT-Vibrationserkennung innerhalb eines elektronischen Implantates (300). Der Schwingungswandler kann an der Gehäuseinnenseite (310) angebracht sein, wobei hier die zum Körperäußeren zeigende Gehäusehälfte bevorzugt ist und gegebenenfalls ein Hinweis am Gehäuse anzubringen ist. Eine andere Option ist die Kombination mit der Programmierspule (320), wobei diese dann derart konstruiert ist, dass eine Schwingung bevorzugt im Spektrum der MRT-Vibrationen erfolgt. Bei ICDs kann der Schwingungswandler auch am Hochspannungstransformator (330) angebracht werden, da dieser ebenfalls eine Spule enthält und so im MRT zur Schwingung angeregt wird. Ist der Schwingungswandler gemäß Abbildung 4 aufgebaut, so kann dieser frei im Inneren des Implantates positioniert werden, da dieser dann selbst in der Lage ist, im MRT eine Schwingung zu erzeugen.

Die Figur 4 zeigt schematisch einen MRT-Lorentz-Sensor wie er als Schwingungswandler eingesetzt werden kann. Dieser besteht aus einem piezoelektrischen Kern (410), umwickelt mit einer Spule (420). Über die mit dem Kern verbundenen Zuleitungen (430) werden die Sensorschwingungen erfasst. Die Resonanzfrequenz des Kerns und Spule ist dabei so dimensioniert, dass eine typische Schwingung im MRT erzielt wird. Insbesondere das Vorhandensein eines sehr starken magnetischen Feldes wird bei der Dimensionierung beachtet. Anstelle des piezoelektrischen Kernmaterials kann auch ein Beschleunigungssensor oder andere adäquate Methoden aus dem Stand der Technik eingesetzt werden.

Die Erfindung ermöglicht es, eine zuverlässige Erkennung eines MRT in elektronischen Implantaten zu realisieren. Es ist damit möglich, die bislang benötigten Umprogrammierungen vor und nach einer MRT-Untersuchung einzusparen. Die Erfindung nutzt die Tatsache, dass hervorgerufen durch die Gradientenfelder und das statische Magnetfeld des MRT Vibrationen ausgelöst werden, die auf der Lorentzkraft beruhen.

Diese Vibrationen treten sowohl an MRT-Gerät selbst (typisches MRT-Geräusch) als auch im Implantat auf und können dort durch konstruktive Maßnahmen gezielt erzeugt werden.

## Patentansprüche

1. Implantierbares medizinisches Gerät (IMD) mit einer Einheit zur Detektion von MRT-Aktivität, **gekennzeichnet dadurch, dass** die Einheit zur Detektion von MRT-Aktivität mindestens einen Schwingungswandler beinhaltet, der durch ein MRT-Gerät hervorgerufene Vibrationen und/oder Schwingungen in ein elektrisches und/oder optisches Signal umwandelt und die Einheit zur Detektion vom MRT-Aktivität auf Basis dieses Signals eine MRT-Aktivität erkennt.

2. IMD nach Anspruch 1, **dadurch gekennzeichnet, dass** basierend auf der Erkennung einer MRT-Aktivität ein Betriebsmodus für das IMD ausgewählt wird, der einen sicheren Betrieb unter MRT-Aktivität erlaubt.

3. IMD nach einem der vorangegangenen Ansprüche, **dadurch gekennzeichnet, dass** die Vibrationen durch Lorentz-Kräfte über elektromagnetische Felder vom MRT-Gerät auf den Schwingungswandler übertragen werden und/oder dass die Vibrationen durch Lorentz-Kräfte über elektromagnetische Felder vom MRT-Gerät auf das IMD und von dort auf den Schwingungswandler übertragen werden und/oder dass die Vibrationen im MRT-Gerät entstehen und diese mechanisch auf den Schwingungswandler übertragen werden, wobei ein Schwingungswandler für unterschiedliche Übertragungsarten auslegbar ist und/oder separate Schwingungswandler für die jeweils unterschiedlichen Übertragungsarten vorsehbar sind.

4. IMD nach einem der vorangegangenen Ansprüche, **dadurch gekennzeichnet, dass** zumindest ein Schwingungswandler auf das für MRT-Geräte typische Frequenzspektrum von MRT-Geräuschen abgestimmt ist.

5. IMD nach einem der vorangegangenen Ansprüche, **dadurch gekennzeichnet, dass** zumindest ein Schwingungswandler auch als Beschleunigungssensor nutzbar ist und dieser Beschleunigungssensor für die ratenadaptive Stimulation eines Herzschrittmachers und/oder Defibrillators/Kardioverters und/oder kardialen Resynchronisationsgerätes verwendbar ist.

6. IMD nach einem der vorangegangenen Ansprüche, **dadurch gekennzeichnet, dass** der oder die Schwingungswandler in einem heliumdichten und/oder evakuierten Gehäuse untergebracht sind.

7. IMD nach einem der vorangegangenen Ansprüche, **dadurch gekennzeichnet, dass** zumindest ein Schwingungswandler nach dem Prinzip der Lorentzkraft immer nur dann in Schwingungen versetzbar ist, wenn gleichzeitig ein Magnetfeld mit einer vorbestimmbaren Mindestfeldstärke vorhanden ist und durch magnetische Wechselfelder Wirbelströme induziert werden.

8. IMD nach einem der vorangegangenen Ansprüche, **dadurch gekennzeichnet, dass** zumindest ein Schwingungswandler mechanisch mit einem Hochspannungstransformator verbunden ist.

9. IMD nach einem der vorangegangenen Ansprüche, **dadurch gekennzeichnet, dass** zumindest ein Schwingungswandler mit einer Programmierspule des IMD verbindbar ist, so dass die Programmierspule durch MRT-Aktivität in Schwingungen versetzbar ist, diese Schwingungen auf den Schwingungswandler übertragbar sind und das Resonanzverhalten der Programmierspule an typische MRT-Aktivitäten anpassbar ist.

10. IMD nach einem der vorangegangenen Ansprüche, **dadurch gekennzeichnet, dass** zumindest ein Schwingungswandler aus einem piezoelektrischen Kern umgeben von einer Spule besteht, wobei die Spule durch Lorentzkräfte den piezoelektrischen Kern in Schwingungen versetzt und so ein piezoelektrisches Signal erzeugt, das als Signal zur Detektion von MRT-Aktivität verwendbar ist und/oder dass zumindest ein Schwingungswandler aus einem Beschleunigungssensor als Kern, der von einer Spule umgeben ist, besteht, wobei der Kern von der Spule durch Lorentzkräfte in Schwingungen versetzbar ist.

11. IMD nach einem der vorangegangenen Ansprüche, **dadurch gekennzeichnet, dass** zumindest ein Schwingungswandler typische MRT-Geräusche mittels zumindest eines Mikrophons und/oder zumindest eines Beschleunigungssensor wahrnimmt, die wahrgenommenen MRT-Geräusche filterbar sind und anschließend durch Autokorrelation in einem digitalen Signalprozessor typischen Sequenzen von MRT-Geräuschen zuordenbar sind.

12. IMD nach einem der vorangegangenen Ansprüche, **dadurch gekennzeichnet, dass** zumindest die Signale des Schwingungswandlers mittels eines für die Erkennung von MRT-Geräuschen angepassten Stimmenidentifikationsverfahren und/oder eines neuronalen Netzwerkes verarbeitet werden.

13. IMD nach einem der vorangegangenen Ansprüche, **dadurch gekennzeichnet dass** zumindest eine der folgenden Maßnahmen Teil eines sicheren Betriebszustands bei MRT-Aktivität sind:
- das Wechseln in einen MRI-sicheren Zustand,
- ein verlängertes Verbleiben in einem MRI-sicheren oder gegenüber elektromagnetischen Störfeldern unempfindlichen Zustand,
- die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit, neben der Störung auch Informationen auf diese Weise zu übertragen und auf dem Bildschirm des MRI sichtbar zu machen, und
- die Abgabe einer Therapie und/oder Detektion von elektrischen Zuständen des Gewebes nur in Zeitfenstern erlaubt ist, in denen keine elektromagnetischen Störfelder erkannt werden und/oder dass eine Rekonstruktion einer Messungen für die Bereiche durchgeführt wird, in denen die Detektion aufgrund von erkannten elektromagnetischen Störfeldern nicht erlaubt ist.

14. IMD nach einem der vorangegangenen Ansprüche, **dadurch gekennzeichnet, dass** die Einheit zur Detektion MRT-Aktivität mindestens einen der folgenden Sensoren oder Indikatoren umfasst:
- GMR-Sensor,
- MagFET-Sensor,
- Hallsensor,
- elektrooptische Wandler als Indikator,
- die Überwachung von Batteriespannungen während Kondensatorlade-prozessen als Indikator,
- die Detektion von RF-Feldern als Indikator,
- die Detektion von magnetischen Grandientenfeldern als Indikator, und
- die Detektion von durch elektromagnetische Felder induzierten Strömen als Indikator.

15. Verfahren zur Detektion von MRT-Aktivität, **dadurch gekennzeichnet, dass** ein Schwingungswandler durch ein MRT-Gerät hervorgerufene Vibrationen und/oder Schwingungen in ein elektrisches und/oder optisches Signal umwandelt und eine Einheit zur Detektion vom MRT-Aktivität auf Basis dieses Signals eine MRT-Aktivität erkennt.
